(19) Europäisches Patentamt
European Patent Office
Office européen des brevets

(11) Publication number: **0 312 245 A2**

(12) **EUROPEAN PATENT APPLICATION**

(21) Application number: 88309270.2

(22) Date of filing: 05.10.88

(51) Int. Cl.⁴: C07D 493/08, C07D 487/08, C07C 91/14, A61K 31/34, A61K 31/40, A61K 31/13, //(C07D493/08,307:00,307:00), (C07D487/08,209:00,209:00)

(30) Priority: 09.10.87 ZA 877609

(43) Date of publication of application: 19.04.89 Bulletin 89/16

(84) Designated Contracting States: AT BE CH DE ES FR GB GR IT LI LU NL SE

(71) Applicant: NORISTAN HOLDINGS LIMITED
326 Marks Street
Waltloo Pretoria Transvaal(ZA)

(72) Inventor: Van Der Schyf, Cornelius Johannes
8 Rossini Ave., Van der Hoffpark
Potchefstroom
2520, Transvaal(ZA)
Inventor: Fourie, Theunis Gerhardus
625 Windsor Street, Garsfontein
Pretoria 0002, Transvaal(ZA)
Inventor: Snyckers, Friedrich Otto
117 Clearwater Road, Lynnwood Glen
Pretoria 0002, Transvaal(ZA)
Inventor: Dekker, Theodor Gerhard
23 Borcherd Street, Potchefstroom 2520
Transvaal(ZA)
Inventor: Squier, Gerald James
2 Springside Walk, St. Leonard's on Sea
East Sussex, TB380(GB)
Inventor: Van Der Walt, Jurgens Johannes
No. 123 Wilgeboom Small Holdings, V.d. Hoffpark
Potchefstroom 2520, Transvaal(ZA)
Inventor: Coetzee, William Abraham
12 Constantia Road, Richwood 8001, Cape Town
Cape Province(ZA)
Inventor: Liebenberg, Wilna
32 Rose Garden, Miederpark, Potchefstroom 2520
Transvaal(ZA)

(74) Representative: Froud, Clive et al
ELKINGTON AND FIFE Beacon House 113 Kingsway
London WC2B 6PP(GB)

(54) **Polycyclic compounds and pharmaceutical compositions thereof.**

EP 0 312 245 A2

Xerox Copy Centre

(57) This invention provides compounds of the general formula I:

A-NHR (I)

wherein A is 8,11-oxopentacyclo [5.4.0.$0^{2,6}$.$0^{3,10}$.$0^{5,9}$]undecane optionally substituted with one or more substituents, preferably comprising an alkyl, aryl, hydroxy, keto or amine group, and R is hydrogen or an unsubstituted or substituted aromatic or an unbranched or branched alkyl group, the alkyl group preferably or optionally having as a substituent an aryl or hydroxy group or halogen; and acid addition salts of such compounds.

This invention also provides processes for preparing the above compounds; and calcium antagonists and/or antihypertensive agents containing one or more of the above compounds.

# POLYCYCLIC COMPOUNDS AND PHARMACEUTICAL COMPOSITIONS THEREOF

## BACKGROUND OF THE INVENTION

This invention relates to polycyclic compounds, a process for preparing these compounds, uses and pharmaceutical compositions thereof. More particularly this invention relates to amino derivatives of 8,11-oxopentacylo[5.4.0.0$^{2,6}$.0$^{3,10}$.0$^{5,9}$] undecane, a process for preparing such compounds, their use as calcium antagonists, and pharmaceutical compositions thereof.

Subsequent to the discovery of the antiviral and anti-Parkinsonistic properties of amantadine, considerable research has taken place to exploit the medicinal properties of polycyclic compounds. U.S. Patent 3,449,422 describes certain amino derivatives of pentacyclo[5.4.0.0$^{2,6}$.0$^{3,10}$.0$^{5,9}$] undecane, such as the 8-amino derivatives (A), and their preparation as well as their activity against Asian and swine influenza viruses. In the Europe Patent Application 82306975.2 it has been disclosed that the pentacyclo-undecane amines having structure A furthermore exhibit anti-Parkinsonistic properties.

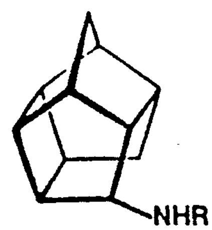

(A)

Antiviral properties have also been reported for a number of amino derivatives of polycyclic hydrocarbons related to A, or the so-called bird-cage type of compounds, for instance in U.S. Patents 3,622,628; 3,536,761; 3,641,148; 3,532,741; 3,456,008 and 3,562,317.

Sasaki et al. (Tetrahedron, 30, 2707, 1974) previously reported (-see the reaction scheme hereunder) that the imino ketone B undergoes reduction with sodium borohydride in tetrahydrofuran/ethanol medium; the hydroxy-aza structure C was assigned to the reaction product. A few years later van der Schyf et al. (Pharmacol. Res. Commun., 18, 407, 1986) reported that the product obtained according to the abovementioned procedure, i.e. by reduction of compound B with sodium borohydride, exhibits calcium antagonistic properties. Interest in the bird-cage type of compounds prompted the present inventors to explore the medicinal properties of aza compounds such as C.

Surprisingly, however, the present inventors established by means of X-ray crystallography that the reduction of the imino ketone B with sodium borohydride, following the procedure described by Sasaki et al., produces the amino ether compound D and not, as previously believed, the hydroxy-aza compound C. The present inventors have therefore found that the structure of the product obtained by the reduction of compound B with sodium borohydride had previously been wrongly assigned.

The formation of the amino ether compound D during the reduction of the imino ketone B with sodium borohydride implies that the ketone group of compound B is attacked initially by the reducing reagent. Nucleophilic attack of the resulting alkoxy ion in E on the imino group produces the ether bridge. It must be understood that the reaction may and probably does proceed through a concerted mechanism. Furthermore, it is also possible that the amino ether D may be in equilibrium with the hydroxy-imine F in certain solvent media. From the $^{13}$C NMR data it could be concluded that it exists in chloroform solution only in the amino ether form D.

Subsequent to the present inventors' finding as mentioned above, A.P. Marchand and co-workers (J. Org. Chem., 53, 2644-2647, 1988) reported their results on the re-investigation of the reduction of imino ketone B with different reducing agents. Firstly, they concluded that the reduction of imino ketone B with sodium borohydride produces only the amino ether D and not the hydroxy-aza compound C; a result which is in accordance with the findings of the present invention. Secondly, Marchand and co-workers have found that the reduction of the imino ketone B with lithium aluminium hydride does not render the amino ether D as the main product, as Sasaki et al. reported; the amino ether D was obtained in a 10% yield, while the amino alcohol G was the main product in 29% yield. The structure of the amino alcohol G was determined by Marchand et al. by means of X- ray crystallographic analysis. In the last instance, Marchand et al. reported a method for the preparation of the hydroxy-aza compound C, which compound Sasaki et al.

believed to have been formed in the reduction of imino ketone B with sodium borohydride. The method entails the reduction of imino ketone B with sodium cyano-borohydride.

From the present invention and the results reported by Marchand et al., the present inventors found that the structures réported by Sasaki et al. for the different reduction products of the imino ketone B are incorrect. Hence neither the structure D of the compound obtained by reduction of imino ketone B with sodium borohydride nor was the pharmacology of structure D known prior to the present invention. In order to illustrate the aforementioned, reference is made to the reaction scheme shown immediately hereunder.

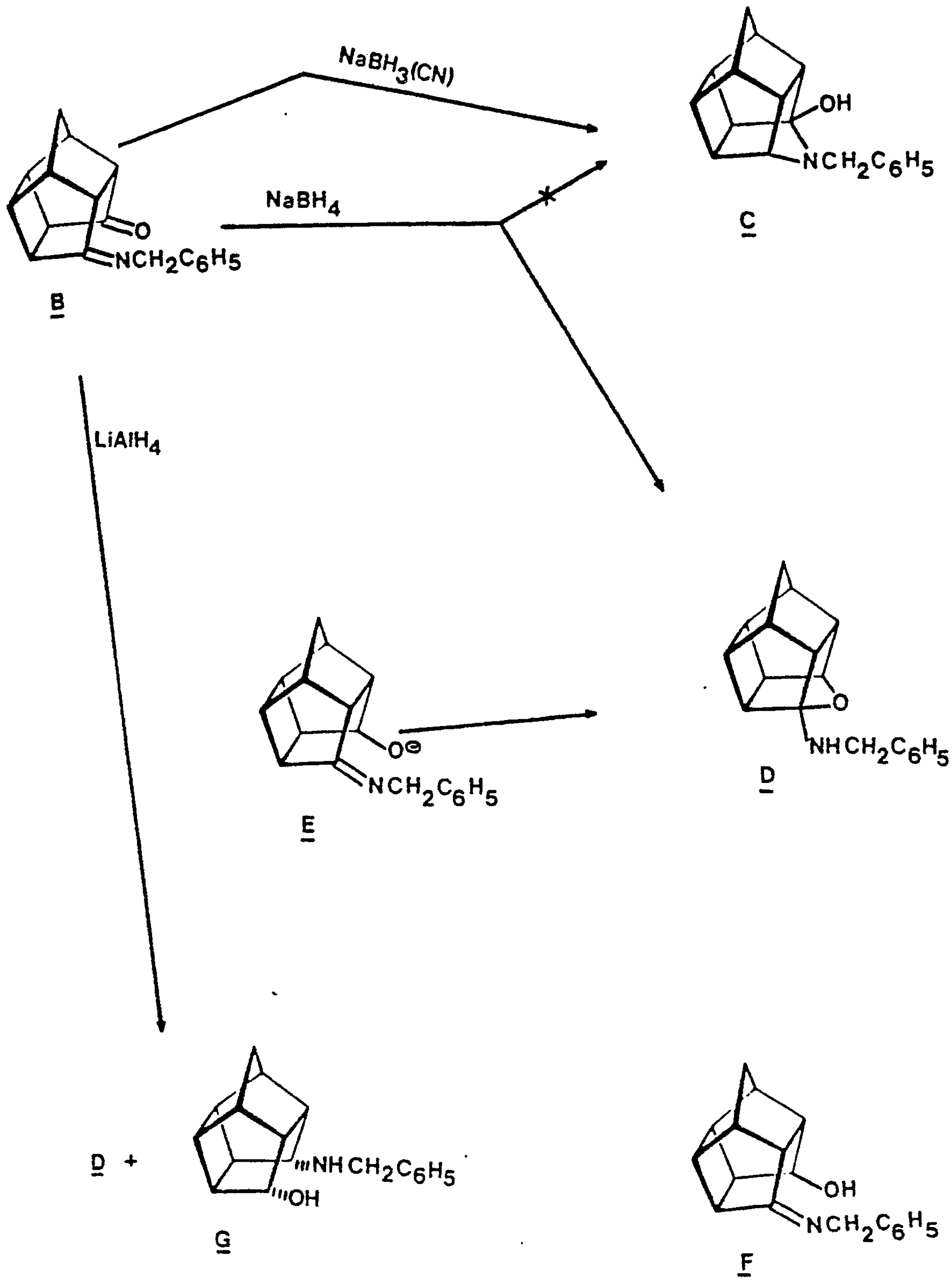


B

C

E

D

D +

G

F

## SUMMARY OF THE INVENTION

According to one aspect of the invention there are provided compounds of the general formula I:

A-NHR (I)

wherein A is 8,11-oxopentacyclo[5.4.0.$0^{2,6}$ .$0^{3,10}$ .$0^{5,9}$]undecane optionally substituted with one or more substituents, preferably comprising an alkyl, aryl, hydroxy, keto or amine group, and R is hydrogen or an unsubstituted or substituted aromatic or an unbranched or branched alkyl group, the alkyl group preferably or optionally having as a substituent an aryl or hydroxy group or halogen; and acid addition salts of such compounds.

According to a preferred aspect of the invention there are provided compounds of the general structural formula II:

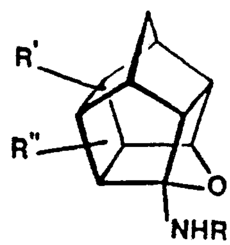

(II)

wherein R is as defined above; and R′ and R″ are as defined for R above; and acid addition salts of such compounds.

Preferred compounds include compounds of the general structural formulae III:

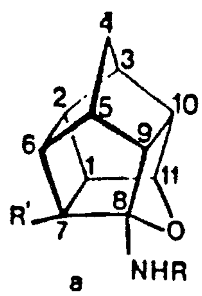

a

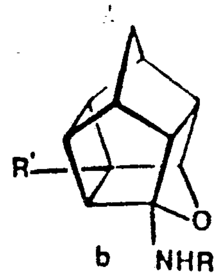

b

(III)

wherein R, and R′ are as defined above; and acid addition salts of such compounds.

Particularly preferred compounds of the above general formulae III are listed in TABLE 1.

TABLE 1

| PARTICULARLY PREFERRED COMPOUNDS | | |
|---|---|---|
| COMPOUND NO. | R | R′ |
| 1 | $CH_3$ | H |
| 2 | $(CH_2)_3CH_3$ | H |
| 3 | $(CH_2)_7CH_3$ | H |
| 4 | $CH_2C_6H_5$ | H |
| 5 | $(CH_2)_2C_6H_5$ | H |
| 6 | $(CH_2)_3C_6H_5$ | H |
| 7 | $CH_3$ | $CH_3$ |
| 8 | $(CH_2)_3CH_3$ | $CH_3$ |
| 9 | $(CH_2)_7CH_3$ | $CH_3$ |
| 10 | $CH_2C_6H_5$ | $CH_3$ |
| 11 | $(CH_2)_2C_6H_5$ | $CH_3$ |
| 12 | $(CH_2)_3C_6H_5$ | $CH_3$ |
| 13 | $CH_2CH_3$ | H |
| 14 | $(CH_2)_{11}CH_3$ | H |
| 15 | $CH_2CH(CH_3)_2$ | H |
| 16 | $(CH_2)_5CH_3$ | H |
| 17 | $C(CH_3)_2CH_2C(CH_3)_3$ | H |
| 18 | $(CH_2)_9CH_3$ | H |
| 19 | $CH_2CH_2OH$ | H |
| 20 | $C_6H_5$ | H |

The compound numbers appearing in TABLE 1 are also used in the examples hereinafter which are given to assist in clearly defining the various compounds according to this invention.

According to another aspect of the present invention there are provided compounds of the general structural formula IV:

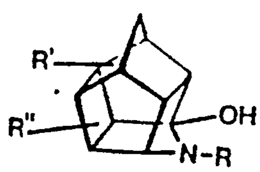

(IV)

wherein R is hydrogen or an unsubstituted or substituted aromatic group or an unbranched or branched alkyl group containing at least two carbon atoms, the alkyl group preferably or optionally having as a substituent an aryl or hydroxy group or halogen, R′ and R″ are as defined for general structural formula II above; and acid addition salts of such compounds.

Preferred compounds of the above structural formula IV include compounds of the general structural formulae V:

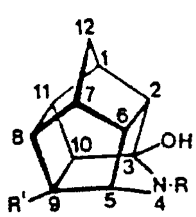

a

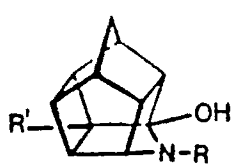

b

(V)

wherein R and R′ are as defined for general structural formula IV above; and acid addition salts of such compounds.

Particularly preferred compounds of structural formulae V above include compounds wherein R and R′ are as defined for compounds 1 to 3 and compounds 5 to 20 in Table 1; and acid addition salts of such compounds.

According to yet another aspect of the present invention there are provided compounds of the general structural formula VI:

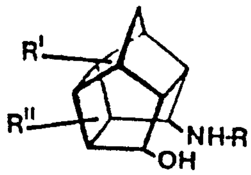

(VI)

wherein R, R′ and R″ are as defined for general structural formula IV above; and acid addition salts of such compounds.

Preferred compounds of the above structural formula VI include compounds of the general structural formulae VII:

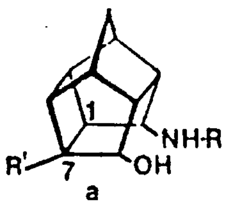

a

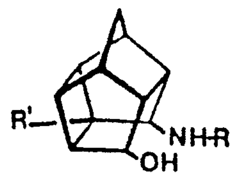

b

(VII)

wherein R and R′ are as defined for general structural formula VI above; and acid addition salts of such compounds.

Particularly preferred compounds of structural formulae VII above include compounds wherein R and R′ are as defined above for structural formulae V; and acid addition salts of such compounds.

The invention extends to positional and optical isomers of the abovementioned compounds.

Also according to the present invention there is provided a process for preparing the compounds of this invention as defined herein, the process including the steps of converting the parent diketone VIII to the corresponding hydroxyamine IX by treatment with the appropriate amine, preferably in tetrahydrofuran in the cold, followed by dehydration of the hydroxyamine IX under mild conditions, preferably by refluxing in dry benzene, to the imino ketone X, which imino ketone is reduced to the amino ether compound III by treatment with a selective reducing agent, such as sodium borohydride.

In the case where R′≠H, the above process may give rise to a mixture of positional isomers (IIIa and IIIb), which may be separated by any method known in the art of organic chemistry.

The compounds of the present invention have an asymmetric structure (III) and will consequently have enantiomeric forms (optical isomers).

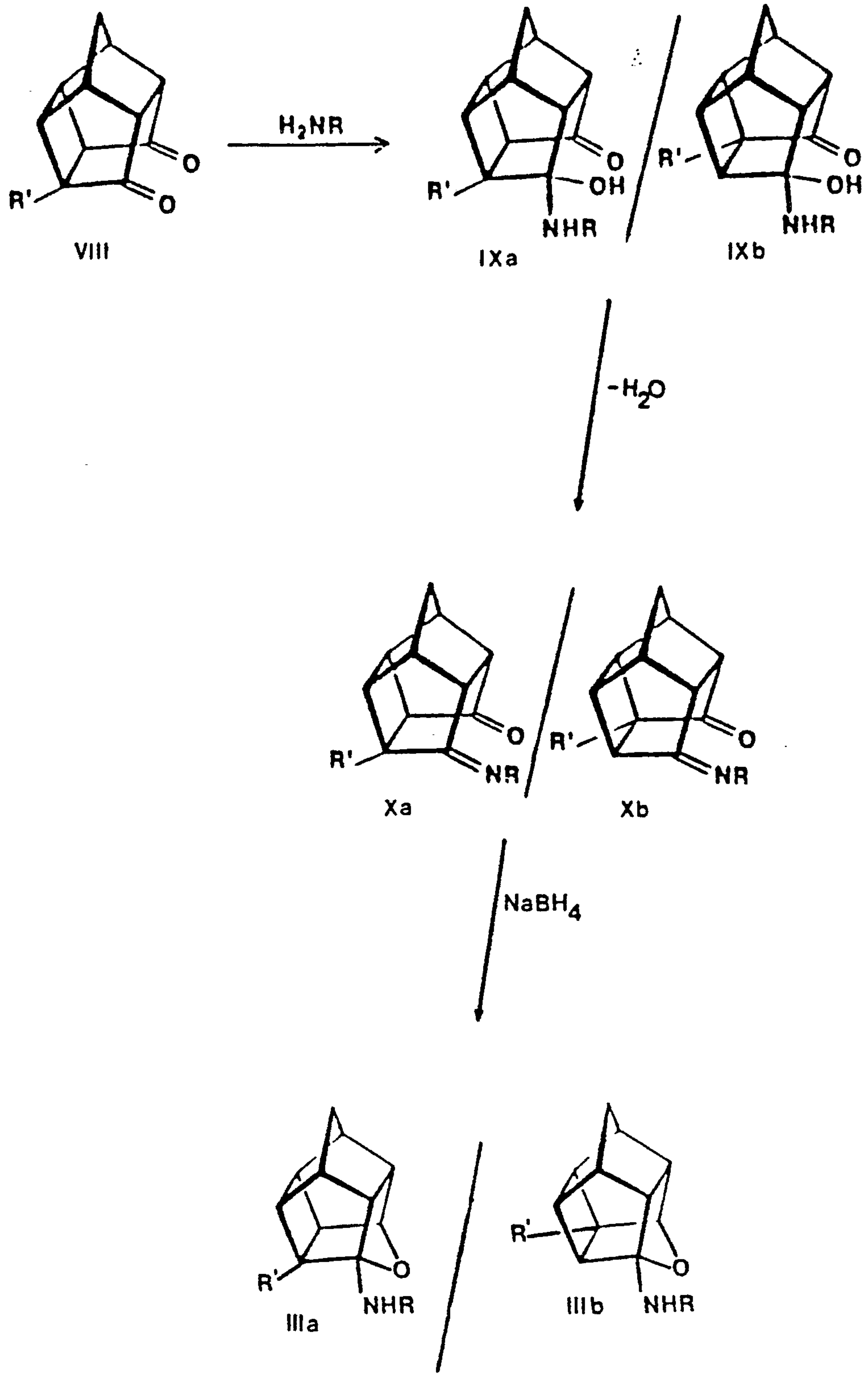

VIII
IXa
IXb
Xa
Xb
IIIa
IIIb

In the case where the unsubstituted diketone (VIII); $R' = H$, which is symmetric and thus optically non-active, is used as a starting material, the process of the invention will give rise to the formation of a racemic mixture of III;$R' = H$. Such enantiomers may be separated by any method known in the art of separating enantiomers. However, when $R' \neq H$, then the parent diketone (VIII;$R' \neq H$) is in itself chiral and the process of this invention may give rise to the formation of four stereo-isomers, namely the two enantiomers of IIIa;$R' \neq H$ and the two enantiomers of IIIb;$R' \neq H$. Whenever a pure enantiomer of VIII;$R' \neq H$ is used, the process will proceed with retention of chirality, i.e. a pure enantiomer of IIIa;$R' \neq H$ or IIIb;$R' \neq H$, or a mixture thereof, will

be formed. It will be understood that the ratio in which the positional isomers IIIa;R′≠H and IIIb;R′≠H are formed from compound VIII;R′≠H is dependent on the reactivity of the carbonyl groups of VIII;R′≠H and the steric influence of group R′ and thus the reaction conditions.

Whenever a mixture of enantiomers and/or diastereomers of the compounds of this invention is produced according to the aforesaid method or any other method, such mixture may be separated into the individual components according to any method known in the art.

Also according to the present invention there is provided a process for separating racemates of the compounds as defined above into their enantiomers. The process includes the steps of converting the racemate (mixture of enantiomers) into diastereomeric N-acyl derivatives by reaction with an optically active acid chloride, preferably R-acetyl mandeloyl chloride, followed by separation of the diastereomeric N-acyl derivatives, by means of any known method in the art, such as column chromatography, flash chromatography or preparative liquid chromatography, followed by hyrolysis of the separated diastereomeric N-acyl derivatives to the corresponding pure enantiomers.

Further details of the process according to the invention will be described in greater detail hereunder.

The invention naturally extends to compounds whenever prepared according to the process of the invention.

The compounds of the abovementioned formulae have generally shown useful calcium antagonistic properties. Voltage clamp experiments of cardiac myocytes have furthermore shown compound 3 to exhibit pronounced frequency dependent block of the calcium current. Antihypertensive activity has also been obtained for compound 3. Accordingly, the invention extends to the use of such compounds as calcium antagonists and as calcium antagonists for cardiac conditions precipitated by uncontrolled repetitive activity and as antihypertensive agents and to such compounds whenever used as calcium antagonists and as calcium antagonists in cardiac tissues where repetitive activity occurs, such as found during supraventricular tachy-arrhythmias and as antihypertensive agents. In other words the invention also extends to at least one compound of the general formulae as defined above as a calcium antagonist or when used as a calcium antagonist for cardiac conditions precipitated by uncontrolled repetitive activity or as an antihypertensive agent and when used as a calcium antagonist.

The invention further extends to calcium antagonists and/or antihypertensive agents comprising one or more compounds of the above general formulae.

According to a further aspect of the invention there are provided pharmaceutical compositions comprising as an active ingredient, a pharmaceutically acceptable amount of at least one pharmacologically acceptable compound of the general formulae as defined above either alone or in admixture with a suitable diluent or adjuvant. The invention extends to methods of preparing such pharmaceutical compositions, and pharmaceutical compositions whenever prepared by such methods.

The invention will now be described in greater detail by means of the following non-limiting examples.

## EXAMPLES

### EXAMPLE 1

8-η-Octylamino-8,11-oxopentacyclo[$5.4.0.0^{2,6}.0^{3,10}.0^{5,9}$]undecane (compound 3)

To a cold, stirred solution of pentacyclo[$5.4.0.0^{2,6}.0^{3,10}.0^{5,9}$]undecan-8,11-dione (VIII;R′=H) (17,4g; 0.1 mol) in tetrahydrofuran (100ml) was added η-octylamine (12,9g; 0.1 mol) over a period of 30 minutes. The reaction mixture was stirred, under ice-cooling, for an addition period of one hour. The resulting precipitate of the hydroxyamine IX;R=$C_8H_{17}$(η);R′=H was filtered, washed with tetrahydrofuran and dried.

Compound IX;R= $C_8H_{17}$(η);R′=H was refluxed in benzene (200ml) under dehydrating conditions, e.g. a Dean-Stark apparatus, until the water of reaction had been completely removed. Evaporation of the benzene in vacuo rendered the imino ketone X;R= $C_8H_{17}$(η);R′=H as a waxy or oily product.

The imino ketone X;R= $C_8H_{17}$(η);R′=H (6g) was reduced by treatment with sodium borohydride (2g) in tetrahydrofuran (40ml) and ethanol (10ml) under refluxing conditions for 5 hours. The solvent was removed in vacuo, water (100ml) was added, and the mixture was extracted with dichloromethane (5 times 50ml). The combined extracts were dried over sodium sulphate and evaporated in vacuo to give compound 3 as an oil, which could be purified by means of chromatography over silica gel. Compound 3 showed the molecular mass in the mass spectrum at m/e 287. The $^{13}C$ NMR spectrum ($CDCl_3$) of compound 3 showed

the following signals (ppm from TMS): 109.8, 82.4, 55.2, 54.8, 44.8, 44.7, 44.5, 43.8, 43.2, 43.0, 41.9, 41.5, 31.8, 31.0, 29.4, 29.2, 27.3, 22.6, and 14.0.

EXAMPLES 2 TO 20

The compounds described under Examples 2 to 20 were prepared according to the procedure outlined in Example 1, by reaction of the appropriate diketone VIII with one molar equivalent of the appropriate amine.

EXAMPLE 2

8-Methylamino-8,11-oxopentacyclo[5.4.0.$0^{2,6}$ .$0^{3,10}$ .$0^{5,9}$ ]-undecane (1) from VIII;R′ = H and methylamine.

EXAMPLE 3

8-η-Butylamino-8,11-oxopentacyclo[5.4.0.$0^{2,6}$ .$0^{3,10}$ .$0^{5,9}$ ]undecane (2) from VIII;R′ = H and η-butylamine : 109.0, 81.6, 54.6, 54.1, 44.8, 44.1, 43.9, 42.7, 42.6, 42.4, 41.3, 40.9, 32.6, 19.8, 13.3.

EXAMPLE 4

8-Benzylamino-8,11-oxopentacyclo[5.4.0.$0^{2,6}$ .$0^{3,10}$ .$0^{5,9}$ ]undecane (4) from VIII;R′ = H and benzylamine. The $^{13}C$ NMR spectrum ($CDCl_3$ ) of compound 4 showed the following signals (ppm from TMS) : 140.8, 128.3 (X 2), 127.7 (X 2), 126.8, 110.0, 82.5, 55.3, 54.8, 47.8, 44.9 (X 2), 44.6, 43.3, 43.2, 42.0, 41.6.

EXAMPLE 5

8-(2-Phenylethyl)-amino-8,11-oxopentacyclo [ 5.4.0.$0^{2,6}$.$0^{3,10}$.$0^{5,9}$ ]undecane (5) from VIII;R′ = H and 2-phenylethylamine.

EXAMPLE 6

8-(3-Phenylpropyl)-amino-8,11-oxopentacyclo [ 5.4.0.$0^{2,6}$.$0^{3,10}$.$0^{5,9}$] undecane (6) from VIII;R′ = H and 3-phenylpropylamine.

EXAMPLE 7

1(or 7)-Methyl-8-methylamino-8,11-oxopentacyclo[5.4.0.$0^{2,6}$.$0^{3,10}$.$0^{5,9}$] undecane (7) from VIII;R′ = $CH_3$ and methylamine.

EXAMPLE 8

1(or7)-Methyl-8-η-butylamino-8,11-oxopentacyclo[5.4.0.$0^{2,6}$ .$0^{3,10}$.$0^{5,9}$]undecane (8) from VIII;R′ = $CH_3$ and η-butylamine.

EXAMPLE 9

1(or7)-Methyl-8-η-octylamino-8,11-oxopentacyclo[5.4.0.$0^{2,6}$.$0^{3,10}$.$0^{5,9}$]undecane (9) from VIII;R′ = $CH_3$ and η-octylamine.

EXAMPLE 10

1(or7)-Methyl-8-benzylamino-8,11-oxopentacyclo [$5.4.0.0^{2,6}.0^{3,10}.0^{5,9}$ ]undecane (10) from VIII;R′ = $CH_3$ and benzylamine.

EXAMPLE 11

1(or7)-Methyl-8-(2-phenylethyl)-amino-8,11-oxopentacyclo [$5.4.0.0^{2,6}.0^{3,10}.0^{5,9}$ ]undecane (11) from VIII;R′ = $CH_3$ and 2-phenylethylamine.

EXAMPLE 12

1(or7)-Methyl-8-(3-phenylpropyl)-amino-8,11-oxopentacyclo[$5.4.0.0^{2,6}.0^{3,10}.0^{5,9}$ ]undecane (12) from VIII;R′ = $CH_3$ and 3-phenylpropylamine.

EXAMPLE 13

8-Ethylamino-8,11-oxopentacyclo [ $5.4.0.0^{2,6}.0^{3,10}.0^{5,9}$]undecane (13) from VIII;R′ = H and ethylamine. The $^{13}C$ NMR spectrum ($CDCl_3$ ) of compound 13 showed the following signals (ppm from TMS) : 109.2, 82.0, 55.0, 54.4, 44.5, 44.3, 44.2, 43.0, 42.7, 41.6, 41.2, 37.7, 15.9.

EXAMPLE 14

8- $\eta$ -Dodecylamino-8,11-oxopentacyclo [$5.4.0.0^{2,6}.0^{3,10}.0^{5,9}$ ]undecane (14) from VIII;R′ = H and $\eta$-dodecylamine. The $^{13}C$ NMR spectrum ($CDCl_3$) of compound 14 showed the following signals (ppm from TMS) : 109.3, 82.0, 55.0, 54.5, 44.5, 44.4, 44.2, 43.4, 43.0, 42.8, 41.7, 41.2, 31.6, 30.8, 29.3 (X 3), 29.2, 29.0, 27.0, 22.4, 13.8.

EXAMPLE 15

8-Isobutylamino-8,11-oxopentacyclo [ $5.4.0.0^{2,6}0^{3,10}.0^{5,9}$]-undecane (15) from VIII;R′ = H and isobutylamine. The $^{13}C$ NMR spectrum ($CDCl_3$ ) of compound 15 showed the following signals (ppm from TMS) : 109.0, 82.1, 54.9, 54.6, 51.5, 44.8, 44.7, 44.4, 43.1, 43.0, 41.8, 41.4, 29.1, 20.4 (X 2).

EXAMPLE 16

8- $\eta$ -Hexylamino-8,11-oxopentayclo [$5.4.0.0^{2,6}.0^{3,10}.0^{5,9}$]undecane (16) from VIII;R′ = H and $\eta$-hexylamine.

EXAMPLE 17

8-*tert*-Octylamino-8,11-oxopentacyclo [$5.4.0.0^{2,6}.0^{3,10}.0^{5,9}$]undecane (17) from VIII;R′ = H and *tert*-octylamine.

EXAMPLE 18

8- $\eta$ -Decylamine-8,11-oxopentacyclo[ $5.4.0.0^{2,6}.0^{3,10}.0^{5,9}$]undecane (18) from VIII;R′ = H and $\eta$-decylamine.

EXAMPLE 19

8-(2-Hydroxyethylamino)-8,11-oxopentacyclo [$5.4.0.0^{2,6}.0^{3,10}.0^{5,9}$] undecane (19) from VIII;R′ = H and ethanolamine. The $^{13}C$ NMR spectrum ($CDCl_3$ ) of compound 19 showed the following signals (ppm from Tms) : 110.0, 82.0, 62.7, 54.8, 54.4, 46.0, 44.6, 44.2, 44.0, 43.0, 42.8, 41.6, 41.2.

## EXAMPLE 20

8-Phenylamino-8,11-oxopentacyclo [ $5.4.0.0^{2,6}.0^{3,10}.0^{5,9}$ ]undecane (20) from VIII;R′ = H and phenylamine. The $^{13}C$ NMR spectrum ($CDCl_3$ ) of compound 20 showed the following signals (ppm from TMS) : 144.5, 128.8 (X 2), 119.1 (X 2), 116.5, 105.5, 82.7, 55.9, 54.5, 44.9, 44.5, 44.4, 43.4, 42.9, 41.6, 41.5.

## EXAMPLE 21

Resolution of 8-$\eta$-butylamino-8,11-oxopentacyclo[$5.4.0.0^{2,6}0^{3,10}.0^{5,9}$] undecane (2).

To a well-stirred mixture of 8- $\eta$ -butylamino-8,11-oxopentacyclo [ $5.4.0.0^{2,6}.0^{3,10}.0^{5,9}$] undecane (2) (4.5g; 19.48 mmol) and an equimolar quantity of sodium bicarbonate in dichloro methane (100ml) was added R-acetyl mandeloyl chloride (4.76g; 22.39 mmol). The reaction mixture was stirred for a period of 30 minutes and filtered. Evaporation of the dichloromethane in vacuo rendered an oily product, consisting mainly of two diastereomeric derivatives of 2, namely the amide derived from the R-enantiomer of 2 and R-acetyl mandelic acid and the amide derived from the S-enantiomer of 2 and R-acetyl mandelic acid. The oily product was dissolved in ether. The ether solution was filtered, washed with a diluted aqueous solution of sodium hydroxide and water, and then dried over anhydrous magnesium sulphate. Evaporation of the ether gave an oil, consisting only of the two diasteriomeric derivatives of 2, mentioned above.

The diastereomers were separated by means of column or flash chromatography over silica gel. The separation was monitored by thin layer chromatography. The $^{13}C$ and $^{1}H$ NMR spectra of the separated diastereomers confirmed a high degree of purity.

Each of the diastereomeric derivatives of 2, prepared and separated according to the procedure described above, could be hydrolised to the corresponding of 2 and R-mandelic acid according to the following procedure. The pure diastereomer (1g) was dissolved in methanol (50ml). A solution of potassium hydroxide (50ml, 5%) was added and the reaction mixture was heated under reflux till the hydrolysis was completed (about 7 hours). The reaction mixture was cooled to room temperature and extracted with dichloro methane (4 x 25ml). The dichloro methane was washed with water, dried over magnesium sulphate and concentrated in vacuo to give the pure enantiomer of 2 as an oil.

The pure enantiomers of 2 showed the following optical properies:

$[\alpha]_D^{19}$ (ethanol)-3.3° and + 3.3°

## EXAMPLE 22

Racemic 8- $\eta$ -octylamino-8,11-oxopentacyclo [$5.4.0.0^{2,6}.0^{3,10}.0^{5,9}$]undecane (3) was separated into its pure enantiomers, $[\alpha]_D^{20}$ -4.1 (ethanol) (oil) and $[\alpha]_D^{20}$ +4.1 (ethanol) (oil), by means of R-acetyl mandeloyl chloride according to the procedure outlined in Example 21.

## EXAMPLE 23

Racemic 8-benzylamino-8,11-oxopentacyclo [$5.4.0.0^{2,6}.0^{3,10}.0^{5,9}$]undecane (4) was separated into its pure enantiomers, $[\alpha]_D^{21}$ -14.3 (ethanol) (crystals, m.p. 77-81° ) and $[\alpha]_D^{21}$ +14.3 (ethanol) (crystals, m.p. 75-79° ), by means of R-acetyl mandeloyl chloride according to the procedure outlined in Example 21.

The structure of compound 4 was determined by means of X-ray crystallographic analysis according to standard procedures. The perspective drawing of 4 is shown below. The fractional coordinates are listed in Table 2.

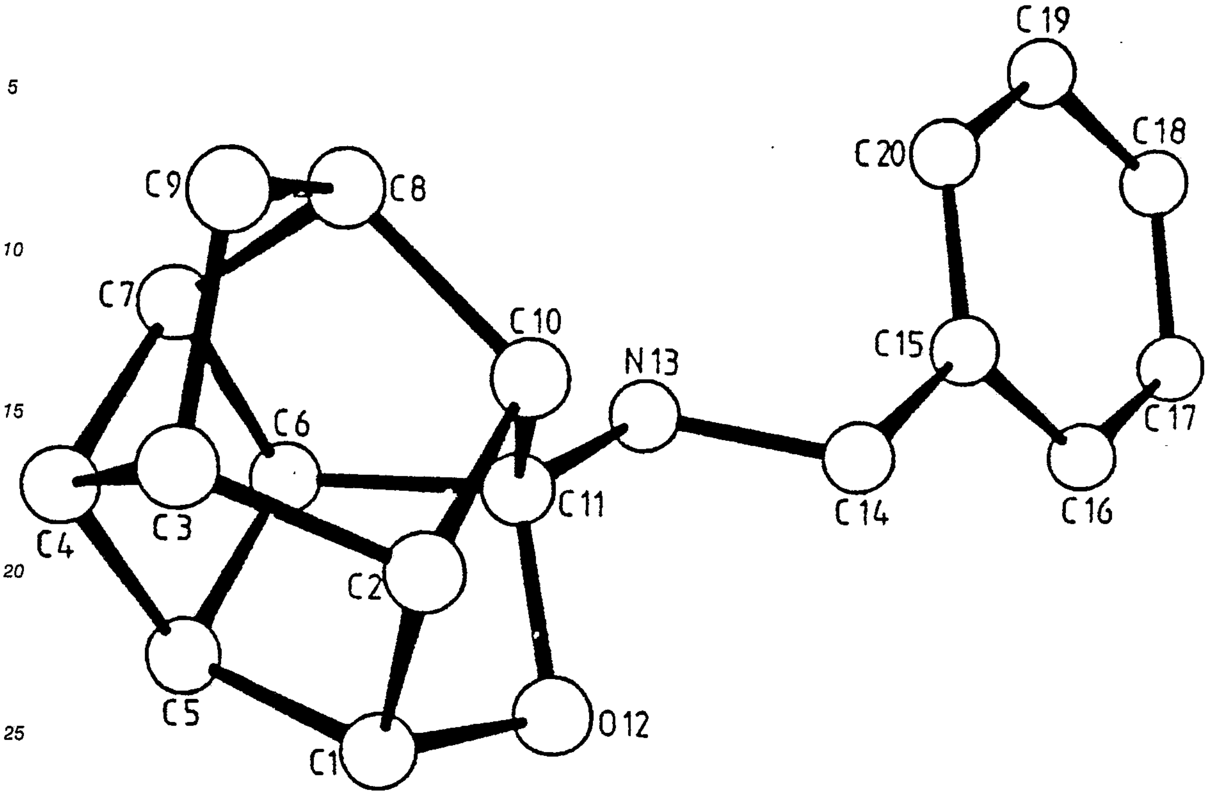

4

TABLE 2

| Fractional coordinates (X10$^4$ ) and equivalent isotropic thermal parameters (X10$^3$ Å$^2$) for Compound 4 . | | | | |
|---|---|---|---|---|
| $\underline{U}_{eq} = \frac{1}{3}\ {}_{ij}\underline{U}_{ij}\underline{a}_i^*\underline{a}_j^*(a_i.a_j)$ | | | | |
| | x/a | y/b | z/c | U $_{eg}$ |
| C(1) | 8076(4) | 1560(4) | 2164 | 66(1) |
| C(2) | 7395(4) | 349(4) | 3200(5) | 63(1) |
| C(3) | 7200(5) | -991(5) | 2481(5) | 78(1) |
| C(4) | 6906(5) | -741(5) | 992(6) | 79(1) |
| C(5) | 7474(4) | 827(4) | 794(5) | 67(1) |
| C(6) | 5978(4) | 573(4) | 858(5) | 55(1) |
| C(7) | -997(4) | -997(4) | 1066(5) | 64(1) |
| C(8) | 4981(4) | -1359(4) | 2586(6) | 75(1) |
| C(9) | 5760(5) | -2140(5) | 3002(6) | 105(2) |
| C(10) | 5881(4) | 93(4) | 3274(6) | 57(1) |
| C(11) | 5954(4) | 1203(4) | 2255(5) | 52(1) |
| C(12) | 7402(2) | 2342(2) | 2434(5) | 63(1) |
| N(13) | 4968(3) | 1634(3) | 2366(5) | 56(1) |
| C(14) | 5099(4) | 2451(4) | 3573(5) | 75(1) |
| C(15) | 3864(4) | 2688(4) | 3684(5) | 55(1) |
| C(16) | 4114(4) | 4042(4) | 3871(6) | 64(1) |
| C(17) | 2999(5) | 4287(5) | 4007(5) | 76(1) |
| C(18) | 1650(5) | 3188(6) | 3987(6) | 87(2) |
| C(19) | 1410(5) | 1855(5) | 3782(5) | 80(1) |
| C(20) | 2494(5) | 1614(4) | 3619(5) | 69(1) |

ELECTROPHYSIOLOGICAL TESTS

Electrophysiological studies were conducted on guinea-pig papillary muscle.

Using a microelectrode technique, action potentials were measured from guinea-pig papillary muscles. Action potentials were evoked by electrical stimulation (platinum electrode, Grass S88 stimulator, 1 Hz stimulation frequency; voltage, 10% above threshold). Perfusion was with a Krebs-Henseleit solution of the following composition (in mmol/l): NaCl 118,5, KCl 4,7, $CaCl_2$ 2,5, $K_2PO_4$, $MgSO_4$ 1,19, $NaHCO_3$ 25, glucose 10; gassed with 95% $O_2$, 5% $CO_2$ 35+1 °C; pH 7,33-7,47. The preparations were electrically driven (pacing intervals 1 s, duration 2-5 ms). Slow ($Ca^{2+}$ mediated) action potentials were evoked in a depolarizing solution (22 mmol/l $K^+$ and 5 mmol/l $Ca^{2+}$) at 0,35 Hz stimulation frequency in order to ensure complete removal of inactivation of slow inward current.

The results of the tests are summarized in Tables 3 and 4 hereunder.

TABLE 3

| Effects of some of the compounds of the invention on the action potential of guinea-pig papillary muscle. | | | | |
|---|---|---|---|---|
| COMPOUND NUMBER | BATH CONCENTRATION (M)[a] | DEGREE OF SUPPRESSION | TIME 1[b] (MIN) | TIME 2[c] (MIN) |
| 1 | $1.20 \times 10^{-4}$ | very little | 25 | - |
| 2 | $1.50 \times 10^{-4}$ | very little | 29 | - |
| 3 | $6.25 \times 10^{-5}$ | complete | 6 | 6 |
| 4 | $2.00 \times 10^{-5}$ | complete | 15 | 10 |
| 6 | $5.00 \times 10^{-5}$ | complete | 18 | 14 |
| 7 | $1.20 \times 10^{-4}$ | very little | 12 | - |
| 8 | $6.00 \times 10^{-5}$ | complete | 22 | 6 |
| 10 | $6.00 \times 10^{-5}$ | complete | 20 | 10 |
| 11 | $1.25 \times 10^{-4}$ | partial | 32 | 35 |
| 12 | $8.00 \times 10^{-5}$ | complete | 12 | 20 |
| Verapamil | $4.00 \times 10^{-5}$ | complete | 10 | 15 |

(a) Lowest concentration of the compound to induce suppression of action potential.
(b) Shortest time needed to attain suppression.
(c) Shortest time needed for reversal of effect by washing out of compound.

TABLE 4

| Effects of the optical isomers of compounds 2 , 3 and 4 on the action potential of guinea-pig papillary muscle. | | | | |
|---|---|---|---|---|
| COMPOUND NUMBER | BATH CONCENTRATION (M)[a] | DEGREE OF SUPPRESSION | TIME 1[b] (MIN) | TIME 2[c] (MIN) |
| 2 | $1.5 \times 10^{-4}$ | little | 25 | - |
| (-) -2 | $1.3 \times 10^{-4}$ | little | 25 | - |
| (+) -2 | $1.3 \times 10^{-4}$ | very little | 25 | - |
| 3 | $6.25 \times 10^{-5}$ | complete | 6 | 6 |
| (-) -3 | $5.23 \times 10^{-5}$ | complete | 10 | 7 |
| (+) -3 | $1.05 \times 10^{-4}$ | incomplete | 30 | 11 |
| 4 | $2.0 \times 10^{-5}$ | complete | 15 | 10 |
| (-) -4 | $5.66 \times 10^{-5}$ | complete | 10 | 5 |
| (+) -4 | $5.66 \times 10^{-5}$ | complete | 20 | 15 |

(a) Lowest concentration of the compound to induce suppression of action potential.
(b) Shortest time needed to attain suppression.
(c) Shortest time needed for reversal of effect by washing out of compound.

## RABBIT EAR PERFUSION TEST

Female New Zealand White rabbits (approximately 4kg) were anaesthetised with Sagatal ® (0.5ml/kg).

The central ear artery was cannulated using a 20 G catheter and the ear cut off at its base. Heparinised (5ml, 200 IU/ml) Krebs bicarbonate solution was perfused through the ear to expel the blood. The ear was placed in a warming jacket at 37° C and attached via the arterial catheter to the perfusion pump. Perfusion of the ear with aerated (95% $O_2$ / 5% $CO_2$)Krebs bicarbonate solution at 37° C was commenced at a rate of 6.5ml/min using a Watson Marlow peristaltic pump. The perfusion pressure was monitored continuously

using a pressure transducer (Type 4-327-1221, Consoliated Electrodynamics) coupled to a Devices Recorder.

Following an equilibration period of 15 minutes, increased tone was induced in the preparation by addition of adrenaline (1µg/ml) to the perfusion fluid.

After a stable tone was observed, various concentrations of test compounds were injected into the perfusion fluid and examined for their effect on the perfusion pressure. The effect of vehicle (0.2ml, 50% Krebs / 50% ethanol) was also tested on the ear preparation.

The results on selected compounds of the present invention obtained with the rabbit ear perfusion test are given in tables 5 - 8.

TABLE 5

| The effect of compounds 3 , 13 , 2 and 15 on the isolated rabbit ear preparation. | | | |
|---|---|---|---|
| Treatment (Compound) | Dose (µg) | Mean % change in perfusion pressure | Mean time taken to return to pre-dose volume (min) |
| Krebs/ethanol | - | - 7.5 | - |
| 3 | 10 | - 8.5 | - |
| | 31.6 | -11.5 | 1.75 |
| | 100 | -16.5 | 4.25 |
| | 316 | -26.0 | 5.5 |
| | 1000 | -41.0 | 8.75 |
| Krebs/ethanol | | - 7.0 | - |
| 13 | 100 | - 6.0 | - |
| | 316 | - 7.0 | - |
| | 1000 | - 5.5 | - |
| | 3160 | -11.5 | 2.0 |
| 2 | 100 | - 7.5 | - |
| | 316 | - 8.0 | - |
| | 1000 | -12.0 | 5.5 |
| | 3160 | -26.0 | 5.75 |
| Krebs/ethanol | | - 7.0 | - |
| 15 | 100 | - 6.5 | - |
| | 316 | - 8.5 | - |
| | 1000 | - 7.0 | - |
| | 3160 | -21.0 | 2.5 |

TABLE 6

A comparison of 3 and Nifedipine on the isolated rabbit ear preparation.

| Treatment | Dose ($\mu g$) | Corrected mean % change in perfusion pressurre | Mean time to return to predose value (min) | Ed25 and 95% fiducial limits ($\mu g$) |
|---|---|---|---|---|
| Nifedipine | 10 | -4 | - | 549 (447-667) |
| | 31.6 | -0.5 | - | |
| | 100 | -9.5 | 3.25 | |
| | 316 | 26 | 5.5 | |
| | 1000 | -39.5 | 6.25 | |
| | 10 | -5.5 | - | |
| | 31.6 | -5.5 | - | |
| 3 | 100 | -11.5 | 4.25 | 600 (468-776) |
| | 316 | -20.5 | 7.0 | |
| | 1000 | -34.5 | 10.5 | |
| | Tissue 1 | Tissue 2 | | |
| Mean % change in perfusion pressure for vehicle | -24 | -5 | | |

TABLE 7

| A comparison of the effects of 3 and 14 on the isolated rabbit ear preparation | | | |
|---|---|---|---|
| Treatment | Dose (μg) | Mean % change in perfusion pressure | Mean time taken to return to predose volume (min) |
| 14 | 31.6 | - 6.1 | - |
| | 100 | -12.4 | - |
| | 316 | - 8.7 | - |
| | 1000 | - 6.6 | - |
| | 3160 | - 7.7 | - |
| 3 | 31.6 | - 9.4 | 6.3 |
| | 100 | -17.3 | 6.3 |
| | 316 | -24.7 | 8.5 |
| | 1000 | -28.9 | 19.3 |
| | 3160 | -27.6 | 15.0 |

TABLE 8

| The effect of compounds 4 and 3 on the isolated rabbit ear preparation | | | |
|---|---|---|---|
| Treatment (Compound) | Dose (μg) | Mean % change in perfusion pressure | Mean time taken to return to predose volume (min) |
| Krebs/ethanol | - | - 7.5 | - |
| 4 | 100 | -16.5 | 1.25 |
| | 316 | -18.0 | 1.0 |
| | 1000 | -14.0 | 1.25 |
| | 3160 | + 4.5 | - |
| Krebs/ethanol | - | - 2.5 | - |
| Krebs | - | - 5.0 | - |
| 3 | 31.6 | - 9.5 | 2.25 |
| | 100 | -19.5 | 4.0 |
| | 316 | -36.0 | 11.5 |
| | 1000 | -41.0 | 10.5 |
| Krebs/ethanol | - | - 6.5 | - |
| Krebs | - | - 8.0 | - |
| Tone was induced in the rabbit ear by perfusion of Krebs bicarbonate solution containing 1 g/kg adrenaline. Values are a mean of two tissues. - indicates a reduction in perfusion pressure + indicates an increase in perfusion pressure | | | |

CALCIUM CURRENT BLOCKADE IN CARDIAC MYOCYTES

EXPERIMENTAL PROCEDURES

Isolation of myocytes.

Ventricular myocytes were isolated from guinea-pig hearts by enzymatic dispersion of the cells using a method related to that employed by Mitra and Morad. After 5 minutes of perfusion at 37 °C on a Langendorff apparatus, the Tyrode solution was changed for a Ca-free solution for 5 minutes, after which the heart was perfused with a 40ml Ca-free solution containing 12mg collagenase (Type II, Sigma) and 10mg protease (Type XIV; Sigma). Perfusion with the soluticn containing enzymes was usually stopped within 5 minutes or as soon as dark areas appeared on the ventricles. The heart was then perfused with a Tyrode solution containing 0.8mM Ca for a few minutes, after which the ventricles were cut in small pieces and the isolated cells were shaken free in a 50ml beaker containing Tyrode solution.

Solutions.

Tyrode solution containing 5.4 mM KCl; 1.8 mM $CaCl_2$ ; 0.5 mM $MgCl_2$; 137.6 mM NaCl; 5 mM glucose and 11.6 mM HEPES. The pH was adjusted to 7.4 with NaOH. During the experiments the temperature was kept constant at room temperature (25 °C). Compound 4 was dissolved in a small volume of 5% HCl and added to Tyrode for a concentration of $2 \times 10^{-5}$M or $10^{-4}$M. No change in the pH of the Tyrode solution could be measured after this addition. The solution for the suction pipette contained 125 mM $CsCl_2$, 5 mM $MgCl_2$,0.5 mM $CaCl_2$, 20 mM TEA-Cl, 4 mM EGTA, 5 mM $Na_2$ ATP and 10 mM HEPES. The pH was adjusted to 7.2 with NaOH. $CsCl_2$ as well as TEA-Cl were used to eliminate potassium currents.

Recording techniques.

Suction pipettes with resistance values between 2 and 5 MΩ were drawn from borosilicate glass (Jencons, H15/10) and heat polished. After formation of a GΩ seal, the patch was ruptured and at least 15 min was allowed for internal dialysis to reach equilibrium. Internal dialysis was indicated by a relatively rapid depolarization after rupturing of the membrane. Voltage clamps were done by using the continuous or chopped clamp method. Sodium currents were eliminated by pre-clamping the membrane at -40 mV before activating calcium currents by clamping the membrane for 400 ms at 0 mV. The results were recorded on magnetic tape or floppy disk. Calculations were done after playback on a Nicolet XF-44 digital oscilloscope. Calcium currents were measured as the difference between the maximum slow inward current and the current recorded after 400 ms. In all experiments pre-drug controls were performed and only preparations which did not show a significant run-down of the calcium current were used for experiments with compound 4.

Results are shown in figures 1 and 2.

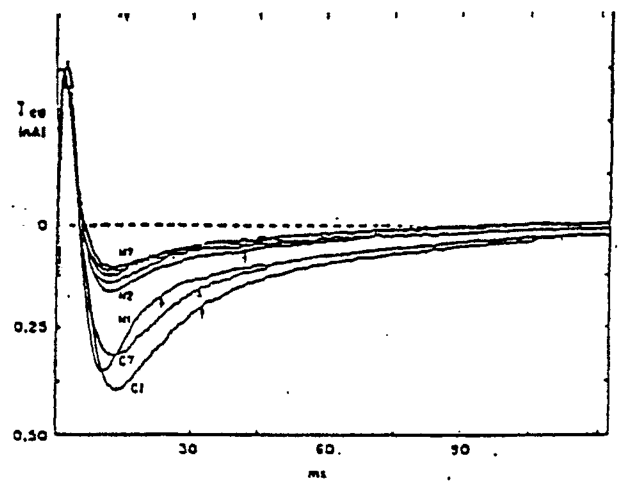


FIG. 1

Fig. 1. Calcium currents recorded in control Tyrode solution (C1 and C7) and in Tyrode containing $1 \times 10^{-4}$ M of compound 4 (N1 to N7). C1 and N1 were obtained as the first calcium currents after a period of

rest, whereas C7 and N7 were the currents obtained after seven successive depolarizations at a rate of 1 Hz. The arrows indicate the points of 50% inactivation.

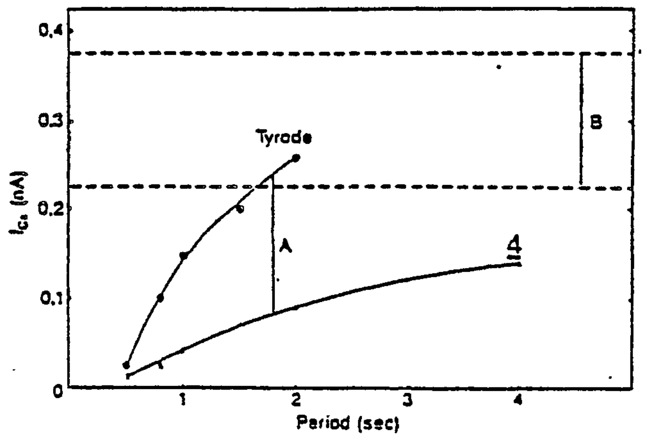


FIG. 2

Fig. 2. The frequency dependence of the calcium current in control solution and with 2 X $10^{-5}$M of compound 4. A = freguency-dependent block, B = freguency-independent block. The horizontal dotted lines represent the steady-state values obtained for the calcium currents determined experimentally with activation periods longer than 1 min.

ANTIHYPERTENSIVE TEST

The effect of Compound 3 on the mean arterial pressure was determined on spontaneously hypertensive rats (SHR ) by means of direct pressure transducing. Groups of 6 male rats of mass 210-300g were used. The average of ten pressure determinations was used. Normotensive (BD-9) rats were used as animal controls, while normal saline solution (containing some traces of polyethylene glycol and ethanol to aid in the solution of the test compounds) was used as solvent and as solvent control.

EXPERIMENTAL PROCEDURE

After verifying the mass of the animal, it was placed under general anaesthesia by administering 60mg/kg pentobarbitone (Sagatal®) intraperitonially. The tracheal tube and carotid artery were both exposed by dissection. An intranule was introduced into the trachea as aid ventilation. The carolid artery was canalated and coupled to a pressure sensor. The sysolic, diastolic and mean arterial pressure were monitored via a Spacelab 514 patient monitor and registered on a Vitatek 551 plotter. During the experiment the exposed tissue of the rat was covered with guaze and kept damp with normal saline.

The drugs and solvent controls were administered intravenously into the dorsal penile vein after stabilization of the blood pressure (BP) had occurred. After drug administration, the BP was recorded every 5 minutes for a period of 30 min.

Results of the experiments are shown in Tables 9 to 13

TABLE 9. Change in mean arterial BP after intravenous administration of 0.9% NaCl solution to SHR's

| RAT | MASS | CONTROL | 5 | 10 | 15 | 20 | 25 | 30 |
|---|---|---|---|---|---|---|---|---|
| | | | Time (min) | | | | | |
| 1 | 255 | 136 | 137 | 134 | 134 | 135 | 133 | 133 |
| 2 | 255 | 178 | 174 | 173 | 167 | 179 | 176 | 177 |
| 3 | 139 | 115 | 111 | 111 | 102 | 102 | 96 | 104 |
| 4 | 245 | 153 | 153 | 146 | 149 | 146 | 142 | 147 |
| 5 | 266 | 160 | 163 | 165 | 161 | 157 | 163 | 159 |
| 6 | 285 | 141 | 140 | 140 | 138 | 137 | 133 | 133 |
| Av. | 258(7) | 147(9) | 146(9) | 145(9) | 142(10) | 143(11) | 141(11) | 142(10) |
| Average % change | | | 1(0.8) | 2(1.1) | 4(1.7) | 4(1.7) | 5(2.6) | 4(1.4) |

Standard error of the mean indicated in brackets.

TABLE 10. Change in mean arterial BP after intravenous administration of Nifedipine (1mg/kg) to BD-9 rats (normatensive)

| RAT | MASS | CONTROL | 5 | 10 | 15 | 20 | 25 | 30 |
|---|---|---|---|---|---|---|---|---|
| | | | Time (min) | | | | | |
| 1 | 260 | 102 | 67 | 65 | 68 | 67 | 63 | 61 |
| 2 | 241 | 90 | 59 | 67 | 66 | 60 | 55 | 57 |
| 3 | 300 | 95 | 80 | 74 | 65 | 62 | 62 | 63 |
| 4 | 287 | 119 | 59 | 69 | 74 | 77 | 80 | 79 |
| 5 | 286 | 126 | 74 | 67 | 63 | 65 | 67 | 60 |
| 6 | 271 | 126 | 60 | 66 | 66 | 64 | 63 | 62 |
| Av. | 274(9) | 110(7) | 67(4) | 68(1) | 67(2) | 66(2) | 65(3) | 64(3) |
| Average % change | | | 38(5.5) | 37(4.4) | 38(3.8) | 39(3.1) | 40(2.8) | 41(3.4) |

Standard error of the mean indicated in brackets.

TABLE 11. Change in mean arterial BP after intravenous administration of Nifedipine (1mg/kg) to SHR's

Time (min)

| RAT | MASS | CONTROL | 5 | 10 | 15 | 20 | 25 | 30 |
|---|---|---|---|---|---|---|---|---|
| 1 | 255 | 135 | 105 | 96 | 95 | 93 | 95 | 95 |
| 2 | 289 | 136 | 67 | 64 | 68 | 69 | 71 | 73 |
| 3 | 245 | 131 | 88 | 104 | 107 | 92 | 93 | 93 |
| 4 | 262 | 169 | 85 | 88 | 93 | 100 | 106 | 110 |
| 5 | 261 | 177 | 80 | 81 | 84 | 83 | 79 | 81 |
| 6 | 256 | 160 | 74 | 74 | 77 | 80 | 84 | 85 |
| Av. | 261(6) | 151(8) | 83(5) | 85(6) | 87(6) | 86(5) | 88(5) | 90(5) |
| Average % change | | | 44(5.4) | 43(6.0) | 41(5.8) | 42(4.2) | 41(4.4) | 40(4.3) |

Standard error of the mean indicated in brackets.

TABLE 12. Change in mean arterial BP after intravenous administration of Compound 4 (10mg/kg) to normatensive BD-9 rats

Time (min)

| RAT | MASS | CONTROL | 5 | 10 | 15 | 20 | 25 | 30 |
|---|---|---|---|---|---|---|---|---|
| 1 | 227 | 104 | 70 | 92 | 85 | 78 | 71 | 68 |
| 2 | 241 | 125 | 100 | 90 | 79 | 88 | 103 | 104 |
| 3 | 280 | 130 | 122 | 116 | 86 | 84 | 85 | 82 |
| 4 | 224 | 129 | 117 | 110 | 107 | 101 | 90 | 89 |
| 5 | 210 | 113 | 118 | 111 | 98 | 85 | 84 | 80 |
| 6 | 251 | 136 | 132 | 129 | 126 | 119 | 110 | 110 |
| Av. | 239(10) | 123(5) | 110(9) | 108(6) | 97(7) | 93(6) | 91(6) | 89(6) |
| Average % change | | | 11(5.4) | 12(3.7) | 21(4.8) | 25(3.1) | 28(2.7) | 28(3.4) |

Standard error of the mean indicated in brackets.

TABLE 13. Change in mean arterial BP after intravenous administration of Compound 4 (10mg/kg) to SHR's

| RAT | MASS | CONTROL | Time (min) 5 | 10 | 15 | 20 | 25 | 30 |
|---|---|---|---|---|---|---|---|---|
| 1 | 240 | 147 | 136 | 139 | 132 | 122 | 96 | 77 |
| 2 | 222 | 162 | 147 | 157 | 140 | 142 | 144 | 143 |
| 3 | 232 | 162 | 116 | 109 | 80 | 67 | 73 | 89 |
| 4 | 241 | 171 | 158 | 145 | 140 | 141 | 96 | 95 |
| 5 | 260 | 163 | 155 | 108 | 79 | 75 | 73 | 72 |
| 6 | 227 | 165 | 150 | 141 | 131 | 105 | 88 | 82 |
| Av. | 237(6) | 162(3) | 144(6) | 133(8) | 117(12) | 109(13) | 95(11) | 93(11) |
| Average % change | | | 11(3.5) | 18(5.4) | 28(7.6) | 33(8.2) | 41(6.8) | 43(6.4) |

Standard error of the mean indicated in brackets.

In respect of pharmaceutical compositions, one or more of the above suitable compounds may be incorporated in a pharmaceutical composition for administration to a human or animal patient. The method of preparing such composition includes the steps of ensuring that the compound(s) are free of undesirable impurities - this may require repeated re-crystallisation, or washing; comminuting the compound(s) to a required particle size; and incorporating and providing the compound(s) in a desired form together with a suitable adjuvant or diluent for administration to a patient for example in solid (powder, tablet or capsule form), or liquid form (injectable or liquid medicine) for internal application, for example in a suspension or cream, or in a (dissolved) jelly form.

Although the invention in its various aspects has been described above in certain preferred embodiments, it will be readily apparent to any person skilled in the art that various modifications and/or variations of the invention are possible. Such modifications and/or variations of the invention are to be considered as forming part of the invention and as falling within the scope of the invention as herein described.

## Claims

1. Compounds of the general formula I:

A-NHR (I)

wherein A is 8,11-oxopentacyclo [5.4.0.$0^{2,6}$ .$0^{3,10}$.$0^{5,9}$]undecane optionally substituted with one or more substituents, preferably comprising an alkyl, aryl, hydroxy, keto or amine group, and R is hydrogen or an unsubstituted or substituted aromatic or an unbranched or branched alkyl group, the alkyl group preferably or optionally having as a substituent an aryl or hydroxy group or halogen; and acid addition salts of such compounds.

2. Compounds of the general structural formula II:

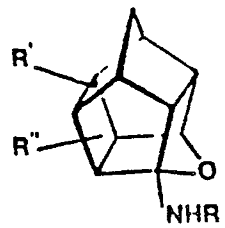

(II)

wherein R, R′ and R″ are as defined for R in claim 1; and acid addition salts of such compounds.

3. Compounds of the general structural formulae III:

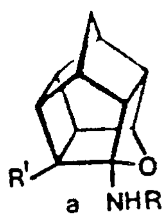

a

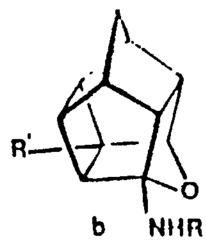

b

(III)

wherein R, and R′ are as defined for R in claim 1; and acid addition salts of such compounds.

4. 8-η-Octylamino-8,11-oxopentacyclo[5.4.0.$0^{2,6}$.$0^{3,10}$.$0^{5,9}$]undecane
5. 8-Methylamino-8,11-oxopentacyclo[5.4.0.$0^{2,6}$.$0^{3,10}$.$0^{5,9}$]undecane
6. 8-η-Butylamino-8,11-oxopentacyclo[5.4.0.$0^{2,6}$.$0^{3,10}$.$0^{5,9}$]undecane
7. 8-Benzylamino-8,11-oxopentacyclo[5.4.0.$0^{2,6}$.$0^{3,10}$.$0^{5,9}$]undecane
8. 8-(2-Phenylethyl)-amino-8,11-oxopentacyclo[5.4.0.$0^{2,6}$.$0^{3,10}$.$0^{5,9}$]undecane
9. 8-(3-Phenylpropyl)-amino-8,11-oxopentacyclo[5.4.0.$0^{2,6}$.$0^{3,10}$.$0^{5,9}$]undecane
10. 1(or 7)-Methyl-8-methylamino-8,11-oxopentacyclo[5.4.0.$0^{2,6}$.$0^{3,10}$.$0^{5,9}$]undecane
11. 1(or 7)-Methyl-8-η-butylamino-8,11-oxopentacyclo [5.4.0.$0^{2,6}$.$0^{3,10}$.$0^{5,9}$]undecane
12. 1(or 7)-Methyl-8-η-octylamino-8,11-oxopentacyclo [5.4.0.$0^{2,6}$.$0^{3,10}$.$0^{5,9}$]undecane
13. 1(or 7)-Methyl-8-benzylamino-8,11-oxopentacyclo[5.4.0.$0^{2,6}$.$0^{3,10}$.$0^{5,9}$]undecane
14. 1(or 7)-Methyl-8-(2-phenylethyl)-amino-8,11-oxopentacyclo[5.4.0.$0^{2,6}$.$0^{3,10}$.$0^{5,9}$]undecane
15. 1(or 7)-Methyl-8-(3-phenylpropyl)-amino-8,11-oxopentacyclo[5.4.0.$0^{2,6}$.$0^{3,10}$.$0^{5,9}$]undecane
16. 8-Ethylamino-8,11-oxopentacyclo[5.4.0.$0^{2,6}$.$0^{3,10}$.$0^{5,9}$]undecane
17. 8-η-Dodecylamino-8,11-oxopentacyclo[5.4.0.$0^{2,6}$.$0^{3,10}$.$0^{5,9}$]undecane
18. 8-Isobutylamino-8,11-oxopentacyclo[5.4.0.$0^{2,6}0^{3,10}$.$0^{5,9}$]undecane
19. 8-η-Hexylamino-8,11-oxopentayclo[5.4.$0^{2,6}$ .$0^{3,10}$.$0^{5,9}$]undecane
20. 8-*tert*-Octylamino-8,11-oxopentacyclo[5.4.0.$0^{2,6}$.$0^{3,10}$.$0^{5,9}$]undecane
21. 8-η-Decylamino-8,11-oxopentacyclo[5.4.0.$0^{2,6}$.$0^{3,10}$.$0^{5,9}$]undecane
22. 8-(2-Hydroxyethylamino)-8,11-oxopentacyclo [5.4.0.$0^{2,6}$.$0^{3,10}$.$0^{5,9}$]undecane
23. 8-Phenylamino-8,11-oxopentacyclo[5.4.0.$0^{2,6}$.$0^{3,10}$.$0^{5,9}$]undecane
24. Compounds of the general structural formula IV:

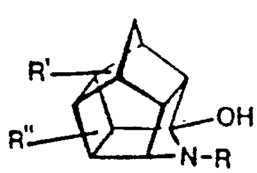

(IV)

wherein R is hydrogen or an unsubstituted or substituted aromatic group or an unbranched or branched alkyl group containing at least two carbon atoms, the alkyl group preferably or optionally having as a substituent an aryl or hydroxy group or halogen, R′ and R″ are as defined for R in claim 1; and acid addition salts of such compounds.

25. Compounds of the general structural formulae V:

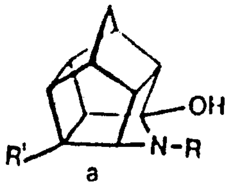
a

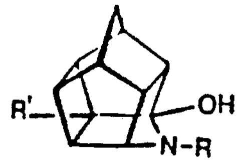
b

(V)

wherein R and $R'$ are as defined for R in claim 1; and acid addition salts of such compounds.

26. Compounds of general structural formulae V, wherein R and $R'$ are as defined for R in claim 1 except for the compound when R is benzyl and $R'$ is hydrogen; and acid addition salts of such compounds.

27. 4-$\eta$-Octyl-4-azahexacyclo [ $5.4.1.0^{2,6}.0^{3,10}.0^{5,9}.0^{8,11}$]dodecan-3-ol

28. 4-Methyl-4-azahexacyclo [ $5.4.1.0^{2,6}.0^{3,10}.0^{5,9}.0^{8,11}$]dodecan-3-ol

29. 4- $\eta$ -Butyl-4-azahexacyclo [ $5.4.1.0^{2,6}.0^{3,10}.0^{5,9}.0^{8,11}$]dodecan-3-ol

30. 4-(2-Phenylethyl)-4-azahexacyclo [ $5.4.1.0^{2,6}.0^{3,10}.0^{5,9}.0^{8,11}$] dodecan-3-ol

31. 4-(3-Phenylpropyl)-4-azahexacyclo [ $5.4.1.0^{2,6}.0^{3,10}.0^{5,9}.0^{8,11}$] dodecan 3-0l

32. 9(or 10),4-Dimethyl-4-azahexacyclo [ $5.4.1.0^{2,6}.0^{3,10}.0^{5,9}.0^{8,11}$] dodecan-3-ol

33. 9(or 10)-methyl-4-$\eta$ -butyl-4-azahexacyclo [ $5.4.1.0^{2,6}.0^{3,10}.0^{5,9}.0^{8,11}$]dodecan-3-ol

34. 9(or 10)-Methyl-4- $\eta$ -octyl-4-azahexacyclo [ $5.4.1.0^{2,6}.0^{3,10}.0^{5,9}.0^{8,11}$]dodecan-3-ol

35. 9(or 10)-Methyl-4-benzyl -4-azahexacyclo [ $5.4.1.0^{2,6}.0^{3,10}.0^{5,9}.0^{8,11}$] dodecan-3-ol

36. 9(or 10)-Methyl-4-(2-phenylethyl)-4-azahexacyclo[$5.4.1.0^{2,6}.0^{3,10}.0^{5,9}.0^{8,11}$] dodecan-3-ol

37. 9(or 10)-Methyl-4-(3-phenylpropyl)-4-azahexacyclo-[$5.4.1.0^{2,6}.0^{3,10}.0^{5,9}.0^{8,11}$] dodecan-3-ol

38. 4-Ethyl-4-azahexacyclo [$5.4.1.0^{2,6}.0^{3,10}.0^{5,9}.0^{8,11}$]dodecan-3-ol

39. 4-Dodecyl-4-azahexacyclo[ $5.4.1.0^{2,6}.0^{3,10}.0^{5,9}.0^{8,11}$]dodecan-3-ol

40. 4-Isolbutyl-4-azahexacyclo[ $5.4.1.0^{2,6}.0^{3,10}.0^{5,9}.0^{8,11}$]dodecan-3-ol

41. 4-$\eta$-Hexyl-4-azahexacyclo [$5.4.1.0^{2,6}.0^{3,10}.0^{5,9}.0^{8,11}$]dodecan-3-ol

42. 4-*tert*-Octyl-4-azahexacyclo [ $5.4.1.0^{2,6}.0^{3,10}.0^{5,9}.0^{8,11}$] dodecan-3-ol

43. 4-$\eta$-Decyl-4-azahexacyclo [$5.4.1.0^{2,6}.0^{3,10}.0^{5,9}.0^{8,11}$]dodecan-3-ol

44. 4-(2-Hydroxyethyl)-4-azahexacyclo [$5.4.1.0^{2,6}.0^{3,10}.0^{5,9}.0^{8,11}$] dodecan-3-ol

45. 4-Phenyl-4-azahexacyclo [$5.4.1.0^{2,6}.0^{3,10}.0^{5,9}.0^{8,11}$]dodecan-3-ol

46. Compounds of the general structural formula VI:

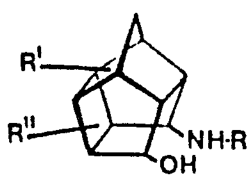

(VI)

wherein R, $R'$ and $R''$ are as defined for R in claim 1; and acid addition salts of such compounds.

47. Compounds of the general structural formulae VII:

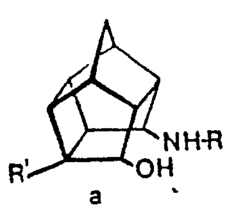
a

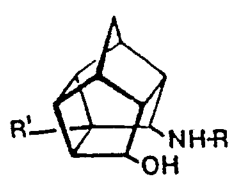
b

(VII)

wherein R and $R'$ are as defined for R in claim 1; and acid addition salts of such compounds.

48. Compounds of general structural formulae VII wherein R and $R'$ are as defined for R in claim 1 except for the compound when R is benzyl and $R'$ is hydrogen; and acid addition salts of such compounds.

49. 11-$\eta$-Octylaminopentacyclo[ $5.4.0.0^{2,6}.0^{3,10}.0^{5,9}$]undecan-8-ol

50. 11-Methylaminopentacyclo[ $5.4.0.0^{2,6}.0^{3,10}.0^{5,9}$]undecan-8-ol

51. 11-$\eta$-Butylaminopentacyclo [$5.4.0.0^{2,6}.0^{3,10}.0^{5,9}$]undecan-8-ol

52. 11-(2-Phenylethyl)aminopentacyclo[ $5.4.0.0^{2,6}.0^{3,10}.0^{5,9}$]undecan-8-ol

53. 11-(3-Phenylpropyl)aminopentacyclo[ $5.4.0.0^{2,6}.0^{3,10}.0^{5,9}$]undecan-8-ol

54. 1(or 7)-Methyl-11-methylaminopentacyclo[ 5.4.0.$0^{2,6}$.$0^{3,10}$.$0^{5,9}$]undecan-8-ol
55. 1(or 7)-Methyl-11-$\eta$-butylaminopentacyclo [5.4.0.$0^{2,6}$.$0^{3,10}$.$0^{5,9}$]undecan-8-ol
56. 1(or 7)-Methyl-11-$\eta$-octylaminopentacyclo [5.4.0.$0^{2,6}$.$0^{3,10}$.$0^{5,9}$]undecan-8-ol
57. 1(or 7)-Methyl-11-benzylaminopentacyclo [5.4.0.$0^{2,6}$.$0^{3,10}$.$0^{5,9}$]undecan-8-ol
58. 1(or 7)-Methyl-11-(2-phenylethyl)aminopentacyclo[5.4.0.$0^{2,6}$.$0^{3,10}$ .$0^{5,9}$]undecan-8-ol
59. 1(or 7)-Methyl-11-(3-phenylpropyl)aminopentacyclo[5.4.0.$0^{2,6}$.$0^{3,10}$ .$0^{5,9}$]undecan-8-ol
60. 11-Ethylaminopentacyclo [5.4.0.$0^{2,6}$.$0^{3,10}$.$0^{5,9}$]undecan-8-ol
61. 11-Dodecylaminopentacyclo [5.4.0.$0^{2,6}$.$0^{3,10}$ .$0^{5,9}$]undecan-8-ol
62. 11-Isobutylaminopentacyclo [5.4.0.$0^{2,6}$.$0^{3,10}$.$0^{5,9}$]undecan-8-ol
63. 11-$\eta$-Hexylaminopentacyclo [5.4.0.$0^{2,6}$.$0^{3,10}$ .$0^{5,9}$]undecan-8-ol
64. 11-*tert*-Octylaminopentacyclo[ 5.4.0.$0^{2,6}$.$0^{3,10}$ .$0^{5,9}$]undecan-8-ol
65. 11-$\eta$-Decylaminopentacyclo[ 5.4.0.$0^{2,6}$.$0^{3,10}$ .$0^{5,9}$]undecan-8-ol
66. 11-(2-Hydroxyethyl)aminopentacyclo[ 5.4.0.$0^{2,6}$.$0^{3,10}$.$0^{5,9}$]undecan-8-ol
67. 11-Phenylaminopentacyclo [5.4.0.$0^{2,6}$.$0^{3,10}$ .$0^{5,9}$]undecan-8-ol
68. Positional and/or optical isomers of the compounds claimed in any one of the preceding claims.
69. Compounds according to claim 68, including:

(-)-8-$\eta$-Butylamino-8,11-oxopentacyclo[5.4.0.$0^{2,6}$.$0^{3,10}$.$0^{5,9}$]undecane
(+)-8-$\eta$-Butylamino-8,11-oxopentacyclo[5.4.0.$0^{2,6}$.$0^{3,10}$.$0^{5,9}$]undecane
(-)-8-$\eta$-Octylamino-8,11-oxopentacyclo [5.4.0.$0^{2,6}$.$0^{3,10}$.$0^{5,9}$undecane
(+)-8-$\eta$-Octylamino-8,11-oxopentacyclo[ 5.4.0.$0^{2,6}$.$0^{3,10}$.$0^{5,9}$]undecane
(-)-8-Benzylamino-8,11-oxopentacyclo[ 5.4.0.$0^{2,6}$.$0^{3,10}$.$0^{5,9}$]undecane
(+)-8-Benzylamino-8,11-oxopentacyclo [ 5.4.0.$0^{2,6}$.$0^{3,10}$.$0^{5,9}$]undecane

70. A process for preparing compounds of the general formulae (I) to (VII), the process including the steps of converting a parent diketone to a corresponding hydroxyamine by treatment with the appropriate amine, preferably in tetrahydrofuran in the cold, followed by dehydration of the hydroxyamine under mild conditions, preferably by refluxing in dry benzene, to an imino ketone which is reduced to an amino ether compound by treatment with a selective reducing agent.

71. A process as claimed in claim 70 including the step of separating racemates of the compounds into their enantiomers.

72. A process as claimed in claim 71, including the steps of converting the mixture of enantiomers into diastereomeric N-acyl derivatives by reaction with an optically active acid chloride, followed by separation of the diastereomeric N-acyl derivatives, by means of any known method in the art, followed by hyrolysis of the separated diastereomeric N-acyl derivatives to the corresponding pure enantiomers.

73. A compound of general formulae (I) to (VII) as claimed in any one of claims 1 to 69, whenever used as a calcium antagonist and/or an antihypertensive agent.

74. A calcium antagonist and/or antihypertensive agent comprising one or more compounds of the general formulae (I) to (VII) as claimed in any one of claims 1 to 69.

75. A pharmaceutical composition comprising as an active ingredient, a pharmaceutically acceptable amount of at least one pharmacologically acceptable compound of the general formulae (I) to (VII) as claimed in any one of claims 1 to 69, either alone or in admixture with a suitable diluent or adjuvant.

76. A method of treating a patient for hypertension, including the step of administering to a patient a suitable amount of at least one pharmacologically acceptable compound of the general formulae (I) to (VII) as claimed in any one of claims 1 to 69, either alone or in admixture with a suitable diluent or adjuvant.

77. A method of treating a patient for angina, Prinzmetal's angina, supra-ventricular arrhythmias or tachy-arrhythmias, including the step of administering to a patient suitable amount of at least one pharmacologically acceptable compound of the general formulae (I) to (VII) as claimed in any one of claims 1 to 69, either alone or in admixture with a suitable diluent or adjuvant.